(19) **Europäisches Patentamt / European Patent Office / Office européen des brevets**

(11) **EP 3 971 760 A1**

(12) # EUROPEAN PATENT APPLICATION
published in accordance with Art. 153(4) EPC

(43) Date of publication:
**23.03.2022 Bulletin 2022/12**

(21) Application number: **20804771.2**

(22) Date of filing: **12.05.2020**

(51) International Patent Classification (IPC):
**G06F 30/27** (2020.01) **G06N 20/00** (2019.01)
**G06N 20/20** (2019.01) **G16C 20/70** (2019.01)

(52) Cooperative Patent Classification (CPC):
**G06F 30/27; G06N 20/00; G06N 20/20;
G16C 20/70**

(86) International application number:
**PCT/JP2020/018967**

(87) International publication number:
**WO 2020/230781 (19.11.2020 Gazette 2020/47)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **16.05.2019 JP 2019092818**

(71) Applicant: DAIKIN INDUSTRIES, LTD.
**Osaka-shi, Osaka 530-8323 (JP)**

(72) Inventors:
• **KAWABE, Rumi**
**Osaka-shi, Osaka 530-8323 (JP)**
• **TAKAKUWA, Tatsuya**
**Osaka-shi, Osaka 530-8323 (JP)**
• **MASUDA, Haruhisa**
**Osaka-shi, Osaka 530-8323 (JP)**
• **KURAMOTO, Kei**
**Osaka-shi, Osaka 530-8323 (JP)**

(74) Representative: **Hoffmann Eitle
Patent- und Rechtsanwälte PartmbB
Arabellastraße 30
81925 München (DE)**

(54) **LEARNING MODEL GENERATION METHOD, PROGRAM, STORAGE MEDIUM, AND PRELEARNED MODEL**

(57) Discovery of a preferable combination of water-repellent agents and the like requires tests, evaluations, and the like conducted repeatedly, resulting in a heavy burden in terms of time and cost. A learning model generation method generates a learning model for determining by using a computer an evaluation of an article in which a surface-treating agent is fixed onto a base material. The learning model generation method includes an obtaining step (S12), a learning step (S15) and a generating step (S16). In the obtaining step (S12), the computer obtains teacher data. The teacher data includes base material information, treatment agent information, and the evaluation of the article. In the learning step (S15), the computer learns on the basis of a plurality of the teacher data obtained in the obtaining step (S12). In the generating step (S16), the computer generates the learning model on the basis of a result of learning in the learning step (S15). The article is obtained by fixing the surface-treating agent onto the base material. The learning model receives input information as an input, and outputs the evaluation. The input information is unknown information different from the teacher data. The input information includes at least the base material information and the treatment agent information.

FIG. 1

**Description**

[Technical Field]

The present disclosure relates to a learning model generation method, a program, a storage medium storing the program, and a learned model.

[Background Art]

Patent Literature 1 (JPA No. 2018-535281) discloses a preferable combination of water-repellent agents.
Patent Literature 2 (JPA No. 1999-511949) discloses an optimization analysis device and a storage medium storing an optimization analysis program.

[Summary of Invention]

[Technical Problem]

Discovery of a preferable combination of water-repellent agents and the like requires tests, evaluations, and the like conducted repeatedly, resulting in a heavy burden in terms of time and cost.

[Solution to Problem]

A learning model generation method according to a first aspect generates a learning model for determining by using a computer an evaluation of an article in which a surface-treating agent is fixed onto a base material. The learning model generation method includes an obtaining step, a learning step, and a generating step. In the obtaining step, the computer obtains teacher data. The teacher data includes base material information, treatment agent information, and the evaluation of the article. The base material information is information regarding a base material. The treatment agent information is information regarding the surface-treating agent. In the learning step, the computer learns on the basis of a plurality of the teacher data obtained in the obtaining step. In the generating step, the computer generates the learning model on the basis of a result of learning in the learning step. The article is obtained by fixing the surface-treating agent onto the base material. The learning model receives input information as an input, and outputs the evaluation. The input information is unknown information different from the teacher data. The input information includes at least the base material information and the treatment agent information.
The learning model thus generated enables evaluation by using a computer, and in turn reduction of extensive time and cost required for conducting the evaluation.
A learning model generation method according to a second aspect includes an obtaining step, a learning step, and a generating step. In the obtaining step, a computer obtains teacher data. The teacher data includes base material information, treatment agent information, and an evaluation. The base material information is information regarding a base material. The treatment agent information is information regarding a surface-treating agent. The evaluation is regarding an article in which the surface-treating agent is fixed onto the base material. In the learning step, the computer learns on the basis of a plurality of the teacher data obtained in the obtaining step. In the generating step, the computer generates the learning model on the basis of a result of learning in the learning step. The article is obtained by fixing the surface-treating agent onto the base material. The learning model receives input information as an input, and outputs the evaluation. The input information is unknown information different from the teacher data. The input information includes at least the base material information and information regarding the evaluation.
A learning model generation method according to a third aspect is the learning model generation method according to the first aspect or the second aspect, in which in the learning step, the learning is performed by a regression analysis and/or ensemble learning that is a combination of a plurality of regression analyses.
A program according to a fourth aspect is a program with which a computer determines, by using a learning model, an evaluation of a base material onto which a surface-treating agent is fixed. The program includes an input step, a determination step, and an output step. In the input step, the computer receives input information as an input. In the determination step, the computer determines the evaluation. In the output step, the computer outputs the evaluation determined in the determination step. The article is obtained by fixing the surface-treating agent onto the base material. The learning model learns, as teacher data, base material information, which is information regarding the base material, treatment agent information, which is information regarding the surface-treating agent to be fixed onto the base material, and the evaluation. The input information is unknown information different from the teacher data, including the base material information and the treatment agent information.
A program according to a fifth aspect is a program with which a computer determines, by using a learning model, treatment agent information that is optimal for fixation onto a base material. The program includes an input step, a determination step, and an output step. In the input step, the computer receives input information as an input. In the determination step, the computer determines the treatment agent information that is optimal. In the output step, the computer outputs the treatment agent information that is optimal determined in the determination step. The learning model learns, as teacher data, base material information, treatment agent information, and an evaluation. The base material information is information regarding a base material. The treatment agent information is information regarding a surface-treating agent. The evaluation is regarding an article in which the surface-treating agent is fixed onto the base material. The treatment agent information is information regarding a surface-treating agent to be fixed onto the base material. The input information is unknown information different from the teacher data. The input information includes at least the base material information and information regarding the evaluation. The article is obtained by fixing the surface-treating agent onto the base material.

A program according to a sixth aspect is the program according to the fourth aspect or the fifth aspect, in which the evaluation is any of water-repellency information, oil-repellency information, antifouling property information, or processing stability information. The water-repellency information is information regarding water-repellency of the article. The oil-repellency information is information regarding oil-repellency of the article. The antifouling property information is information regarding an antifouling property of the article. The processing stability information is information regarding processing stability of the article.

A program according to a seventh aspect is the program according to any of the fourth aspect to the sixth aspect, in which the base material is a textile product.

A program according to an eighth aspect is the program according to the seventh aspect, in which the base material information includes information regarding at least a type of the textile product and a type of a dye. The treatment agent information includes information regarding at least a type of a monomer constituting a repellent polymer contained in the surface-treating agent, a content of a monomeric unit in the polymer, a content of the repellent polymer in the surface-treating agent, a type of a solvent and a content of the solvent in the surface-treating agent, and a type of a surfactant and a content of the surfactant in the surface-treating agent.

A program according to a ninth aspect is the program according to the eighth aspect, in which the teacher data includes environment information during processing of the base material. The environment information includes information regarding any of temperature, humidity, curing temperature, or processing speed during the processing of the base material. The base material information further includes information regarding any of a color, a weave, basis weight, yarn thickness, or zeta potential of the textile product. The treatment agent information further includes information regarding any item of: a type and a content of an additive to be added to the surface-treating agent; pH of the surface-treating agent; or zeta potential thereof.

A program according to a tenth aspect is a storage medium storing the program according to any of the fourth aspect to the ninth aspect.

A learned model according to an eleventh aspect is a learned model for causing a computer to function. The learned model performs calculation based on a weighting coefficient of a neural network with respect to base material information and treatment agent information being input to an input layer of the neural network. The learned model outputs water-repellency information or oil-repellency information of a base material from an output layer of the neural network on the basis of a result of the calculation. The base material information is information regarding the base material. The treatment agent information is information regarding a surface-treating agent. The weighting coefficient is obtained through learning of at least the base material information, the treatment agent information, and an evaluation as teacher data. The evaluation is regarding the article in which the surface-treating agent is fixed onto the base material. The article is obtained by fixing the surface-treating agent onto the base material.

A learned model according to a twelfth aspect is a learned model for causing a computer to function. The learned model performs calculation based on a weighting coefficient of a neural network with respect to base material information and information regarding an evaluation being input to an input layer of the neural network. The learned model outputs treatment agent information that is optimal for a base material from an output layer of the neural network on the basis of a result of the calculation. The base material information is information regarding the base material. The weighting coefficient is obtained through learning of at least the base material information, the treatment agent information, and the evaluation as teacher data. The treatment agent information is information regarding a surface-treating agent to be fixed onto the base material. The evaluation is regarding an article in which the surface-treating agent is fixed onto the base material. The article is obtained by fixing the surface-treating agent onto the base material.

[Brief Description of Drawings]

[Figure 1] Figure 1 shows a configuration of a learning model generation device.

[Figure 2] Figure 2 shows a configuration of a user device.

[Figure 3] Figure 3 shows an example of a decision tree.

[Figure 4] Figure 4 shows an example of a feature space divided by the decision tree.

[Figure 5] Figure 5 shows an example of an SVM

[Figure 6] Figure 6 shows an example of a feature space.

[Figure 7] Figure 7 shows an example of a neuron model in a neural network.

[Figure 8] Figure 8 shows an example of a neural network.

[Figure 9] Figure 9 shows an example of teacher data.

[Figure 10] Figure 10 is a flow chart of an operation of the learning model generation device.

[Figure 11] Figure 11 is a flow chart of an operation of the user device.

[Description of Embodiment]

A learning model according to an embodiment of the present disclosure is described hereinafter. Note that the embodiment described below is a specific example which does not limit a technical scope of the present disclosure, and may be modified as appropriate without departing from the spirit of the present disclosure.

(1) Summary

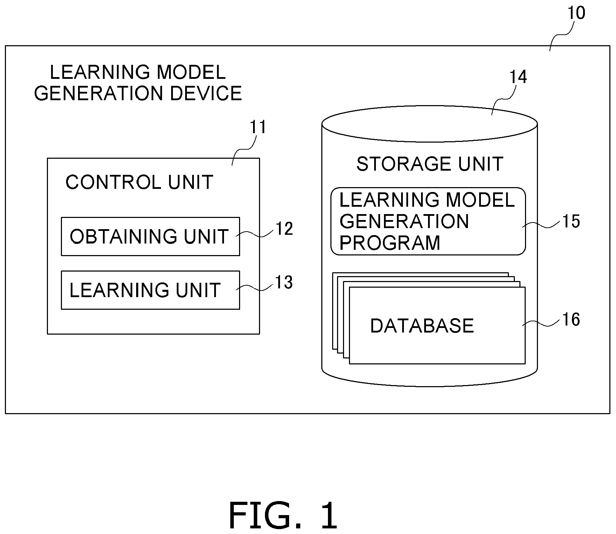

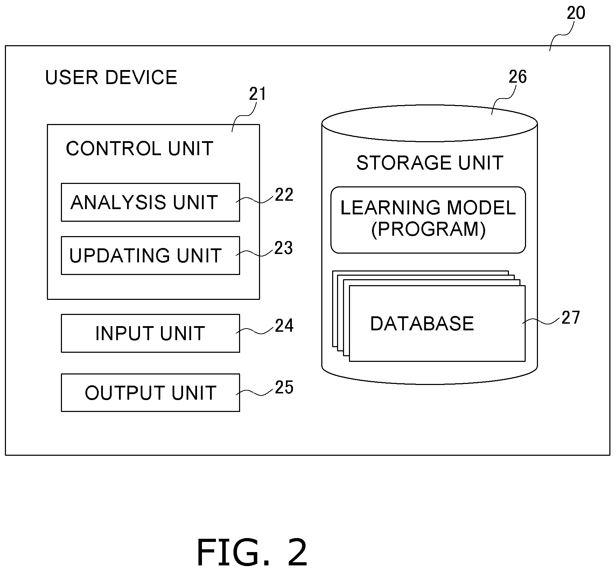

Figure 1 is a diagram showing a configuration of a learning model generation device. Figure 2 is a diagram showing a configuration of a user device.

The learning model is generated by a learning model generation device 10, which is at least one computer, obtaining and learning teacher data. The learning model thus generated is, as a so-called learned model: implemented to a general-purpose computer or terminal; downloaded as a program or the like; or distributed in a state of being stored in a storage medium, and is used in a user device 20, which is at least one computer.

The learning model is capable of outputting a correct answer for unknown information different from the teacher data. Furthermore, the learning model can be updated so as to output a correct answer for various types of data being input.

(2) Configuration of learning model generation device 10

The learning model generation device 10 generates a learning model to be used in the user device 20 described later.

The learning model generation device 10 is a device having a function of a so-called computer. Alternatively, the learning model generation device 10 may include a communication interface such as an NIC and a DMA controller, and be capable of communicating with the user device 20 and the like through a network. Although the learning model generation device 10 is illustrated in Figure 1 as one device, the learning model generation device 10 is preferably compatible with cloud computing. Consequently, in terms of a hardware configuration, the learning model generation device 10 is not required to be accommodated in one housing or provided as a single device. For example, the learning model generation device 10 is configured in such a way that hardware resources thereof are dynamically connected and disconnected according to a load.

The learning model generation device 10 includes a control unit 11 and a storage unit 14.

(2-1) Control unit 11

The control unit 11 is, for example, a CPU and controls the learning model generation device 10 as a whole. The control unit 11 causes each of function units described below to function appropriately, and executes a learning model generation program 15 stored in advance in the storage unit 14. The control unit 11 includes the function units such as an obtaining unit 12, a learning unit 13.

In the control unit 11, the obtaining unit 12 obtains teacher data that is input to the learning model generation device 10, and stores the teacher data thus obtained in a database 16 built in the storage unit 14. The teacher data may be either directly input to the learning model generation device 10 by a user of the learning model generation device 10, or obtained from another device or the like through a network. A manner in which the obtaining unit 12 obtains the teacher data is not limited. The teacher data is information for generating a learning model achieving a learning objective. As used herein, the learning objective is any of: to output an evaluation of an article in which a surface-treating agent is fixed onto a base material; or to output treatment agent information that is optimal for fixation onto the base material. Details thereof are described later.

The learning unit 13 extracts a learning dataset from the teacher data stored in the storage unit 14, to automatically perform machine learning. The learning dataset is a set of data, whose correct answer to an input is known.

The learning dataset to be extracted from the teacher data is different depending on the learning objective. The learning by the learning unit 13 generates the learning model.

(2-2) Machine learning

An approach of the machine learning performed by the learning unit 13 is not limited as long as the approach is supervised learning that employs the learning dataset. A model or an algorithm used for the supervised learning is exemplified by regression analysis, a decision tree, support vector machine, neural network, ensemble learning, random forest, and the like.

Examples of the regression analysis include linear regression analysis, multiple regression analysis, and logistic regression analysis. The regression analysis is an approach of applying a model between input data (explanatory variable) and learning data (objective variable) through the least-squares method or the like. The dimension of the explanatory variable is one in the linear regression analysis, and two in the multiple regression analysis. The logistic regression analysis uses a logistic function (sigmoid function) as the model.

The decision tree is a model for combining a plurality of classifiers to generate a complex classification boundary. The decision tree is described later in detail.

The support vector machine is an algorithm of generating a two-class linear discriminant function. The support vector machine is described later in detail.

The neural network is modeled from a network formed by connecting neurons in the human nervous system with synapses. The neural network, in a narrow sense, refers to a multi-layer perceptron using the Backpropagation. The neural network is typically exemplified by a convolutional neural network (CNN) and a recurrent neural network (RNN). The CNN is a type of feedforward neural network which is not fully connected (sparsely connected). The neural network is described later in detail.

The ensemble learning is an approach of improving classification performance through combination of a plurality of models. An approach used for the ensemble learning is exemplified by bagging, boosting, and random forest. Bagging is an approach of causing a plurality of models to learn by using bootstrap samples of the learning data, and determining an evaluation of new input data by majority vote of the plurality of models. Boosting is an approach of weighting learning data depending on learning results of bagging, and learning incorrectly classified learning data more intensively than correctly classified learning data. Random forest is an approach of, in the case of using a decision tree as a model, generating a set of decision trees (random forest) constituted of a plurality of weakly correlated decision trees. Random forest is described later in detail.

(2-2-1) Decision tree

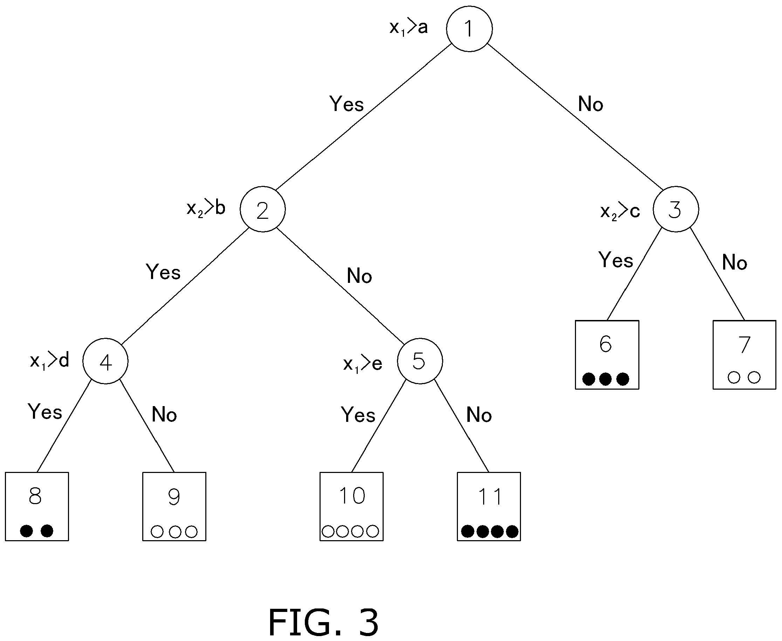

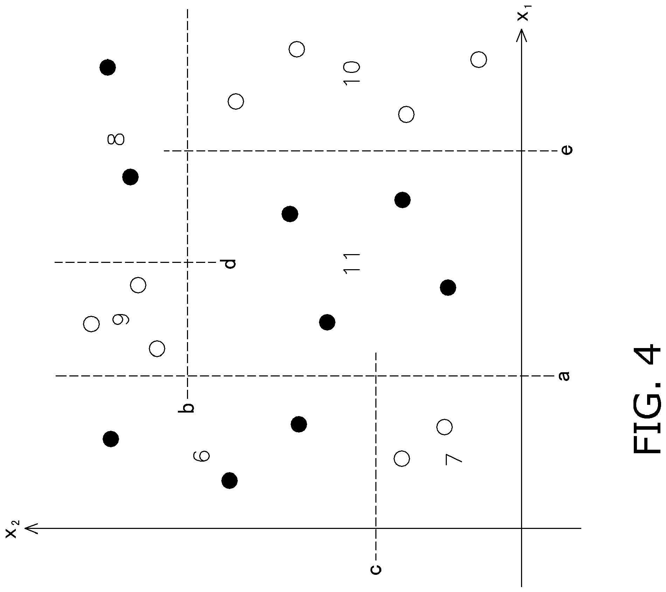

The decision tree is a model for combining a plurality of classifiers to obtain a complex classification boundary (non-linear discriminant function and the like). A classifier is, for example, a rule regarding a magnitude relationship between a value on a specific feature axis and a threshold value. A method for constructing a decision tree from learning data is exemplified by the divide-and-conquer method of repetitively obtaining a rule (classifier) for dividing a feature space into two. Figure 3 shows an example of a decision tree constructed by the divide-and-conquer method. Figure 4 shows a feature space divided by the decision tree of Figure 3. In Figure 4, learning data is indicated by a white dot or a black dot, and each learning data is classified by the decision tree of Figure 3 into a class of white dot or a class of black dot. Figure 3 shows nodes numbered from 1 to 11, and links labeled Yes or No connecting the nodes. In Figure 3, terminal nodes (leaf nodes) are indicated by squares, while non-terminal nodes (root nodes and intermediate nodes) are indicated by circles. The terminal nodes are those numbered from 6 to 11, while the non-terminal nodes are those numbered from 1 to 5. White dots or black dots representing the learning data are shown in each of the terminal nodes. A classifier is provided to each of the non-terminal nodes. The classifiers are rules for determining magnitude relationships between values on feature axis $x_1$, $x_2$ and threshold values a to e. Labels provided to links show determination results of the classifiers. In Figure 4, the classifiers are shown by dotted lines, and regions divided by the classifiers are each provided with the number of the corresponding node.

In the process of constructing an appropriate decision tree by the divide-and-conquer method, consideration of the following three elements (a) to (c) is required.

(a) Selection of feature axis and threshold values for constructing classifiers.
(b) Determination of terminal nodes. For example, the number of classes to which learning data contained in one terminal node belongs. Alternatively, choice of how much a decision tree is to be pruned (how many identical subtrees are to be given to a root node).
(c) Assignment of a class to a terminal node by majority vote.

For example, CART, ID3, and C4.5 are used for learning of a decision tree. CART is an approach of generating a binary tree as a decision tree by dividing a feature space into two at each node except for terminal nodes for each feature axis, as shown in Figure 3 and Figure 4.

In the case of learning using a decision tree, it is important to divide a feature space at an optimal candidate division point at a non-terminal node, in order to improve classification performance of learning data. A parameter for evaluating a candidate division point of a feature space may be an evaluation function referred to as impurity. Function I(t) representing impurity of a node t is exemplified by parameters represented by following equations (1-1) to (1-3). K represents the number of classes.

[Expression 1]

(a) Error rate at node t

$$I(t) = 1 - \max_i P(C_i|t) \qquad (1\text{-}1)$$

(b) Cross entropy (degree of deviation)

$$I(t) = -\sum_{i=1}^{K} P(C_i|t) ln P(C_i|t) \qquad (1\text{-}2)$$

(c) Gini coefficient

$$I(t) = \sum_{i-1}^{K} \sum_{j \neq i} P(C_i|t) P(C_j|t) = \sum_{i-1}^{K} P(C_i|t)(1 - P(C_i|t)) \qquad (1\text{-}3)$$

In the above equations, a probability $P(Ci|t)$ represents a posterior probability of a class $C_i$ at the node t, i.e., a probability of data in the class $C_i$ being chosen at the node t. The probability $P(C_j|t)$ in the second member of the equation (1-3) refers to a probability of data in the class $C_i$ being erroneously taken as a j-th ($\neq$i-th) class, and thus the second member of the equation represents an error rate at the node t. The third member of the equation (1-3) represents a sum of variances of the probability $P(C_i|t)$ regarding all classes.

In the case of dividing a node with the impurity as an evaluation function, for example, an approach of pruning a decision tree to fall within an allowable range defined by an error rate at the node and complexity of a decision tree.

(2-2-2) Support vector machine

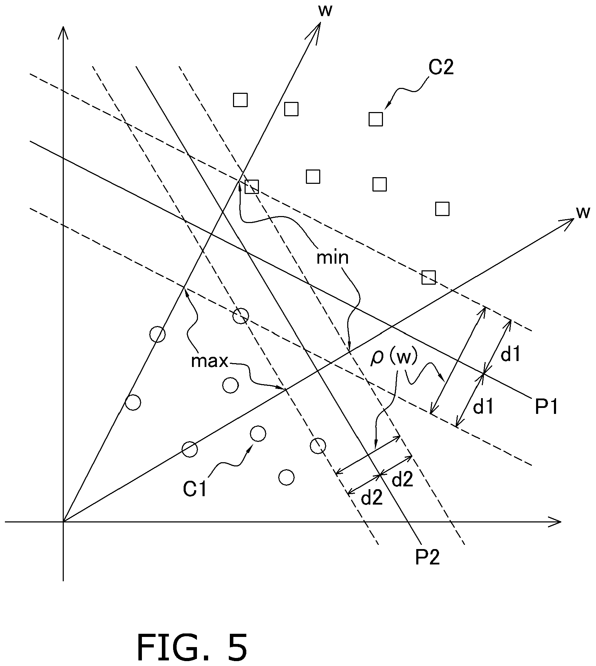

The support vector machine (SVM) is an algorithm of obtaining a two-class linear discriminant function achieving the maximum margin. Figure 5 illustrates the SVM The two-class linear discriminant function refers to, in the feature space shown in Figure 5, classification hyperplanes P1 and P2, which are hyperplanes for linear separation of learning data of two classes C1 and C2. In Figure 5, learning data of the class C1 is indicated by circles, while the learning data of the class C2 is indicated by squares. A margin of a classification hyperplane refers to a distance between the classification hyperplane and learning data closest to the classification hyperplane. Figure 5 shows a margin d1 of the classification hyperplane P1 and a margin d2 of the classification hyperplane P2. The SVM obtains an optimal classification hyperplane P1, which is a classification hyperplane having the maximum margin. The minimum value d1 of a distance between learning data of one class C1 and the optimal classification hyperplane P1 is equal to the minimum value d1 of a distance between learning data of the other class C2 and an optimal classification hyperplane P2.

The following equation (2-1) represents a learning dataset $D_L$ used for the supervised learning of a two-class problem shown in Figure 5.

[Expression 2]

$$D_L = \{(t_i, \boldsymbol{x}_i)\} \, (i = 1, \dots, N) \qquad (2\text{-}1)$$

The learning dataset $D_L$ is a set of pairs of learning data (feature vector) $x_i$ and teacher data ti = {-1, +1}. N represents the number of elements in the learning dataset $D_L$. The teacher data ti indicates to which one of the classes C1 and C2 the learning data $x_i$ belongs. The class C1 is a class of ti = -1, while the class C2 is a class of ti = +1.

A normalized linear discriminant function which holds for all pieces of the learning data $x_i$ in Figure 5 is represented by the following two equations (2-2) and (2-3). A coefficient vector is represented by w, while a bias is represented by b.

[Expression 3]

$$\text{In the case of } t_i = +1 \quad \boldsymbol{w}^T \boldsymbol{x}_i + b \geq +1 \quad (2\text{-}2)$$

$$\text{In the case of } t_i = -1 \quad \boldsymbol{w}^T \boldsymbol{x}_i + b \leq -1 \quad (2\text{-}3)$$

The two equations are represented by the following equation (2-4).
[Expression 4]

$$t_i(\boldsymbol{w}^T \boldsymbol{x}_i + b) \geq 1 \quad (2\text{-}4)$$

In a case in which the classification hyperplanes P1 and P2 are represented by the following equation (2-5), a margin d thereof is represented by the equation (2-6).
[Expression 5]

$$\boldsymbol{w}^T \boldsymbol{x} + b = 0 \quad (2\text{-}5)$$

$$\text{d} = \frac{1}{2}\rho(w) = \frac{1}{2}\left( \min_{x_i \in C_2} \frac{w^T x_i}{\|w\|} - \max_{x_i \in C_1} \frac{w^T x_i}{\|w\|} \right) \quad (2\text{-}6)$$

In the equation (2-6), p(w) represents a minimum value of a difference in length of projection of the learning data $x_i$ of the classes C1 and C2, on a normal vector w of each of the classification hyperplanes P1 and P2. The terms "min" and "max" in the equation (2-6) represent respective points denoted by symbols "min" and "max" in Figure 5. In Figure 5, the optimal classification hyperplane is the classification hyperplane P1 of which margin d is the maximum.

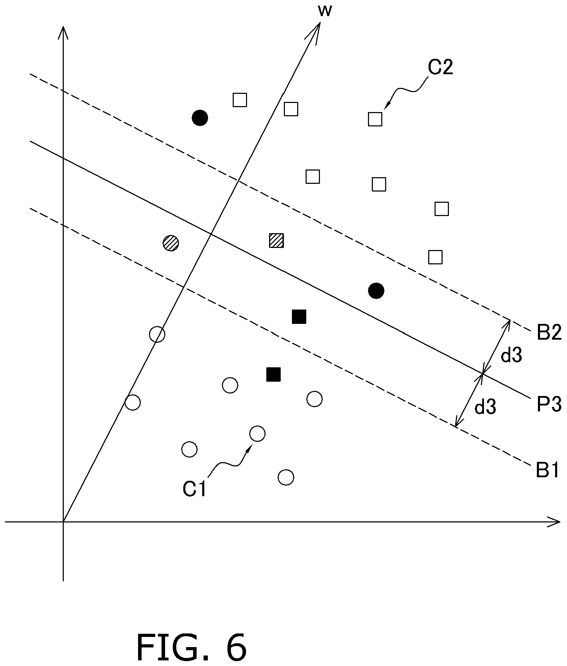

Figure 5 shows a feature space in which linear separation of learning data of the two classes is possible. Figure 6 shows a feature space similar to that of Figure 5, in which linear separation of learning data of the two classes is not possible. In the case in which linear separation of learning data of the two classes is not possible, the following equation (2-7) obtained by expanding the equation (2-4) by introducing a slack variable $\xi_i$ can be used.
[Expression 6]

$$t_i(\boldsymbol{w}^T \boldsymbol{x}_i + b) - 1 + \xi_i \geq 0 \quad (2\text{-}7)$$

The slack variable $\xi_i$ is used only during learning and has a value of at least 0. Figure 6 shows a classification hyperplane P3, margin boundaries B1 and B2, and a margin d3. An equation for the classification hyperplane P3 is identical to the equation (2-5). The margin boundaries B1 and B2 are hyperplanes spaced apart from the classification hyperplane P3 by the margin d3.
When the slack variable $\xi_i$ is 0, the equation (2-7) is equivalent to the equation (2-4). In this case, as indicated by open circles or open squares in Figure 6, the learning data $x_i$ satisfying the equation (2-7) is correctly classified within the margin d3. In this case, a distance between the learning data $x_i$ and the classification hyperplane P3 is greater than the margin d3.
When the slack variable $\xi_i$ is greater than 0 and no greater than 1, as indicated by a hatched circle or a hatched square in Figure 6, the learning data $x_i$ satisfying the equation (2-7) is correctly classified, beyond the margin boundaries B1 and B2, and not beyond the classification hyperplane P3. In this case, a distance between the learning data $x_i$ and the classification hyperplane P3 is less than the margin d3.
When the slack variable $\xi_i$ is greater than 1, as indicated by filled circles or filled squares in Figure 6, the learning data $x_i$ satisfying the equation (2-7) is beyond the classification hyperplane P3 and incorrectly classified.
By thus using the equation (2-7) to which the slack variable $\xi_i$ is introduced, the learning data $x_i$ can be classified even in the case in which linear separation of the learning data of two classes is not possible.
As described above, a sum of the slack variables $\xi_i$ of all pieces of the learning data $x_i$ represents the upper limit of the number of pieces of the learning data $x_i$ incorrectly classified. Here, an evaluation function $L_p$ is defined by the following equation (2-8).
[Expression 7]

$$L_p(\boldsymbol{w}, \xi) = \frac{1}{2}\boldsymbol{w}^T\boldsymbol{w} + C\sum_{i=1}^{N}\xi_i \qquad (2\text{-}8)$$

A solution (w,ξ) that minimizes an output value of the evaluation function $L_p$ is to be obtained. In the equation (2-8), a parameter C in the second expression represents strength of a penalty for incorrect classification. The greater parameter C requires a solution further prioritizing reduction of the number of incorrect classifications (second expression) over reduction of the norm of w (first expression).

(2-2-3) Neural network

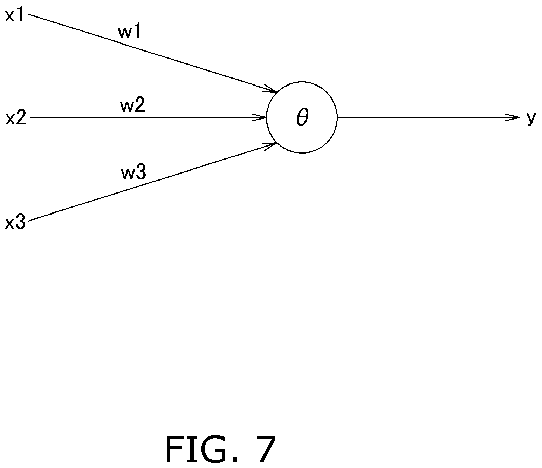

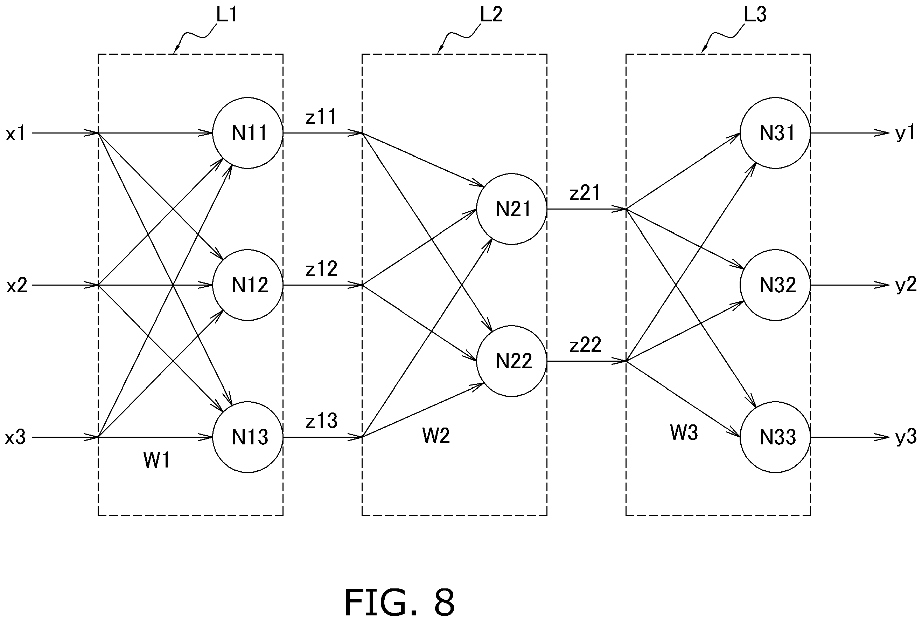

Figure 7 is a schematic view of a model of a neuron in a neural network. Figure 8 is a schematic view of a three-layer neural network constituted by combining the neuron shown in Figure 7. As shown in Figure 7, the neuron outputs an output y for a plurality of inputs x (inputs x1, x2, and x3 in Figure 7). Each of the inputs x (inputs x1, x2 and x3 in Figure 7) is multiplied by a corresponding weight w (weight w1, w2 and w3 in Figure 7). The neuron outputs the output y by means of the following equation (3-1).
[Expression 8]

$$y = \varphi(\sum_{i=1}^{n} x_i w_i - \theta) \qquad (3\text{-}1)$$

In the equation (3-1), the input x, the output y and the weight w are all vectors; θ is a bias; and φ denotes an activation function. The activation function is a non-linear function, for example, a step function (formal neuron), a simple perceptron, a sigmoid function, or a ReLU (ramp function).
The three-layer neural network shown in Figure 8 receives a plurality of input vectors x (input vectors x1, x2 and x3 in Figure 8) from an input side (left side of Figure 8), and outputs a plurality of output vectors y (output vectors y1, y2, and y3 in Figure 8) from an output side (right side of Figure 8). This neural network is constituted of three layers L1, L2, and L3.
In the first layer L1, the input vectors x1, x2 and x3 are multiplied by respective weights, and input to each of three neurons N11, N12, and N13. In Figure 8, W1 collectively denotes the weights. The neurons N11, N12, and N13 output feature vectors z11, z12, and z13 respectively.
In the second layer L2, the feature vectors z11, z12, and z13 are multiplied by respective weights, and input to each of two neurons N21 and N22. In Figure 8, W2 collectively denotes the weights. The neurons N21 and N22 output feature vectors z21 and z22 respectively.
In the third layer L3, the feature vectors z21 and z22 are multiplied by respective weights, and input to each of three neurons N31, N32, and N33. In Figure 8, W3 collectively denotes the weights. The neurons N31, N32, and N33 output output vectors y1, y2, and y3 respectively.
The neural network functions in a learning mode and a prediction mode. The neural network in the learning mode learns the weights W1, W2, and W3 using a learning dataset. The neural network in the prediction mode predicts classification and the like using parameters of the weights W1, W2, and W3 thus learned.
Learning of the weights W1, W2, and W3 can be achieved by, for example, the Backpropagation. In this case, information regarding an error is propagated from the output side toward the input side, in other words, from a right side toward a left side of Figure 8. The Backpropagation learns the weights W1, W2, and W3 with adjustment to reduce a difference between the output y in the case in which the input x is input and the proper output y (teacher data) in each neuron.
The neural network may be configured to have more than three layers. An approach of machine learning with a neural network having four or more layers is known as deep learning.

(2-2-4) Random forest

Random forest is a type of the ensemble learning, reinforcing classification performance through combination of a plurality of decision trees. The learning employing random forest generates a set constituted of a plurality of weakly correlated decision trees (random forest). The following algorithm generates and classifies the random forest.

(A) Repeat the following from m = 1 to m = M.

(a) Generate m bootstrap sample(s) $Z_m$ from N pieces of d-dimensional learning data.
(b) Generate m decision tree(s) by dividing each node t as follows, with $Z_m$ as learning data.

(i) Randomly select d' features from d features (d' < d).
(ii) Determine a feature and a division point (threshold value) achieving the optimal division of the learning data from among the d' features thus selected.
(iii) Divide the node t into two at the division point thus determined.

(B) Output a random forest constituted of m decision tree(s).
(C) Obtain a classification result of each decision tree in the random forest for input data. Majority vote for the classification result of each decision tree determines the classification result of the random forest.

The learning employing random forest enables weakening of correlation between decision trees, through random selection of a preset number of features used for classification at each non-terminal node of the decision tree.

(2-3) Storage unit 14

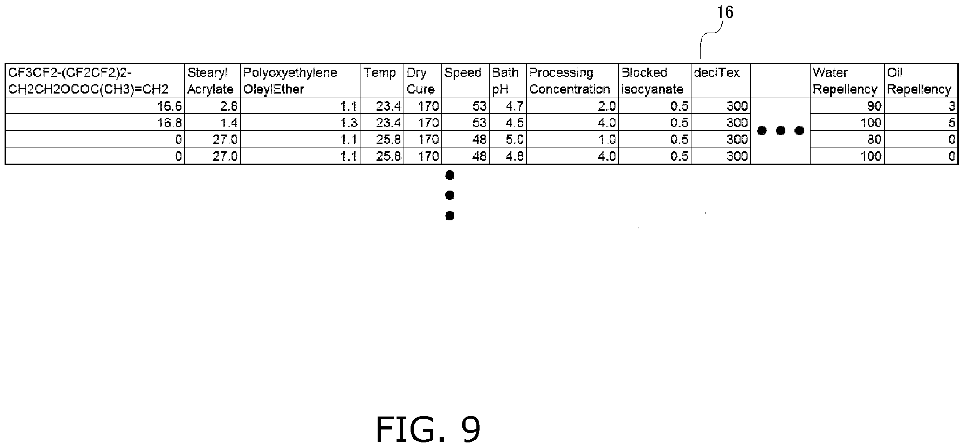

The storage unit 14 shown in Figure 1 is an example of the storage medium and constituted of, for example, flash memory, RAM, a HDD, or the like. The storage unit 14 includes the learning model generation program 15 to be executed by the control unit 11, being stored in advance. The storage unit 14 is provided with the database 16 being built in, in which a plurality of the teacher data obtained by the obtaining unit 12 are stored and appropriately managed. The database 16 stores the plurality of the teacher data as shown in Figure 9, for example. Note that Figure 9 illustrates a part of the teacher data stored in the database 16. The storage unit 14 may also store information for generating a learning model, such as the learning dataset and test data, in addition to the teacher data.

(3) Teacher data

It has been found that the base material information, the treatment agent information, and the evaluation are correlated to each other.
Given this, the teacher data to be obtained for generating the learning model includes at least the base material information, the treatment agent information, and information regarding the evaluation as described below. In light of improving accuracy of an output value, the teacher data preferably further includes environment information. Note that, as a matter of course, the teacher data may also include information other than the following. The database 16 in the storage unit 14 according to the present disclosure shall store a plurality of the teacher data including the following information.

(3-1) Base material information

The base material information is information regarding the base material onto which the surface-treating agent is fixed.
The base material is preferably a textile product. The textile product includes: a fiber; a yarn; a fabric such as a woven fabric, a knitted fabric, and a nonwoven fabric; a carpet; leather; paper; and the like. In the case described hereinafter, the base material is the textile product. Note that the learning model generated in the present embodiment may be used for the base material other than the textile product.
The base material information includes: a type of the textile product; a type of a dye with which a surface of the textile product is dyed; a thickness of fiber used for the textile product; a weave of the fiber; a basis weight of the fiber; a color of the textile product; a zeta potential of the surface of the textile product.
The base material information preferably includes at least information regarding the type of the textile product and/or the color of the textile product, and more preferably further includes information regarding the thickness of the fiber.
Note that the teacher data shown in Figure 9 includes the aforementioned items, which are not illustrated, as the base material information.

(3-2) Treatment agent information

The treatment agent information is information regarding a surface-treating agent to be fixed onto the base material. The surface-treating agent is exemplified by a repellent agent to be fixed onto the base material for imparting water-repellency or oil-repellency thereto. In the case described hereinafter, the surface-treating agent is the repellent agent.
In the present disclosure, the repellent agent preferably contains a repellent polymer, a solvent, and a surfactant.
The repellent polymer is selected from fluorine-containing repellent polymers or non-fluorine repellent polymers. The fluorine-containing repellent polymers and the non-fluorine repellent polymers are preferably acrylic polymers, silicone polymers, or urethane polymers. The fluorine-containing acrylic polymers preferably contain a repeating unit derived from a fluorine-containing monomer represented by the formula CH2=C(-X)-C(=O)-Y-Z-Rf, wherein X represents a hydrogen atom, a monovalent organic group, or a halogen atom; Y represents -O- or -NH-; Z represents a direct bond or a divalent organic group; and Rf represents a fluoroalkyl group having 1 to 6 carbon atoms. The non-fluorine repellent polymers are preferably non-fluorine acrylic polymers containing a repeating unit derived from a long-chain (meth)acrylate ester monomer represented by formula (1) CH2=CA11-C(=O)-O-A12, wherein A11 represents a hydrogen atom or a methyl group; and A12 represents a linear or branched aliphatic hydrocarbon group having 10 to 40 carbon atoms.

The solvent is exemplified by water, or a non-water solvent and the like.

The surfactant is exemplified by a nonionic surfactant, a cationic surfactant, an anion surfactant, an amphoteric surfactant, and the like.

The repellent agent may also include an additive, in addition to the aforementioned components. A type of the additive is exemplified by a cross-linking agent (e.g., blocked isocyanate), an insect repellent, an antibacterial agent, a softening agent, an antifungal agent, a flame retarder, an antistatic agent, an antifoaming agent, a coating material fixative, a penetrating agent, an organic solvent, a catalyst, a pH adjusting agent, a wrinkle-resistant agent, and the like.

The treatment agent information includes a type of a monomer constituting a repellent polymer contained in the surface-treating agent, a content of the monomer in the repellent polymer, a content of the repellent polymer in the surface-treating agent, a type of a solvent and a content of the solvent in the surface-treating agent, and a type of a surfactant and a content of the surfactant in the surface-treating agent.

The treatment agent information preferably includes at least a type of a monomer constituting a repellent polymer contained in the surface-treating agent, and a content of a monomeric unit in the repellent polymer.

The treatment agent information more preferably further includes, in addition to the foregoing, a content of the repellent polymer in the surface-treating agent, a type of a solvent, and a content of the solvent in the surface-treating agent. The treatment agent information still more preferably further includes, in addition to the foregoing, a type of a surfactant and a content of the surfactant in the surface-treating agent.

The treatment agent information may also include information other than the foregoing, such as information regarding a type and a content of an additive to be added to the repellent agent, a pH of the repellent agent, a zeta potential of the repellent agent. As a matter of course, the treatment agent information may include information other than the foregoing. Note that the teacher data shown in Figure 9 includes the aforementioned items, a part of which is omitted in the drawing, as the treatment agent information.

(3-3) Evaluation

The evaluation is information regarding the article in which the surface-treating agent is fixed.

The evaluation includes information regarding chemical properties such as water-repellency information, oil-repellency information, antifouling property information, processing stability information. The evaluation preferably includes at least the water-repellency information and the oil-repellency information. The water-repellency information is information regarding water-repellency of the article after fixation of the surface-treating agent. The water-repellency information is, for example, a value of water-repellency evaluated according to JIS L1092 (spray test). The oil-repellency information is information regarding oil-repellency of the article after fixation of the surface-treating agent. The oil-repellency information is, for example, a value of oil-repellency evaluated according to AATCC 118 or ISO 14419. The antifouling property information is information regarding antifouling property of the article after fixation of the surface-treating agent. The antifouling property information is, for example, a value of antifouling property evaluated according to JIS L1919. The processing stability information is information regarding effects borne by the article and the surface-treating agent, during a step of processing the article after fixation of the surface-treating agent. The processing stability information may have a standard each being defined according to the processing step. For example, the processing stability is indicated by a value obtained by quantifying a degree of adhesion of a resin to a roller that applies pressure to squeeze the textile product.

Note that the teacher data shown in Figure 9 includes as the evaluation at least one of the aforementioned items, which are not illustrated.

(3-4) Environment information

The environment information is regarding an environment in which the surface-treating agent is fixed onto the base material. Specifically, the environment information is regarding, for example, a concentration of the surface-treating agent in a treatment tank, an environment of a factory or the like for performing processing of fixing the surface-treating agent onto the base material, or regarding steps of processing.

The environment information may also include, for example, information regarding a temperature, a humidity, a curing temperature, and a processing speed and the like during the processing of the base material. The environment information preferably includes at least information regarding the concentration of the surface-treating agent in a treatment tank. Note that the teacher data shown in Figure 9 includes the aforementioned items, a part of which is omitted in the drawing, as the environment information.

(4) Operation of learning model generation device 10

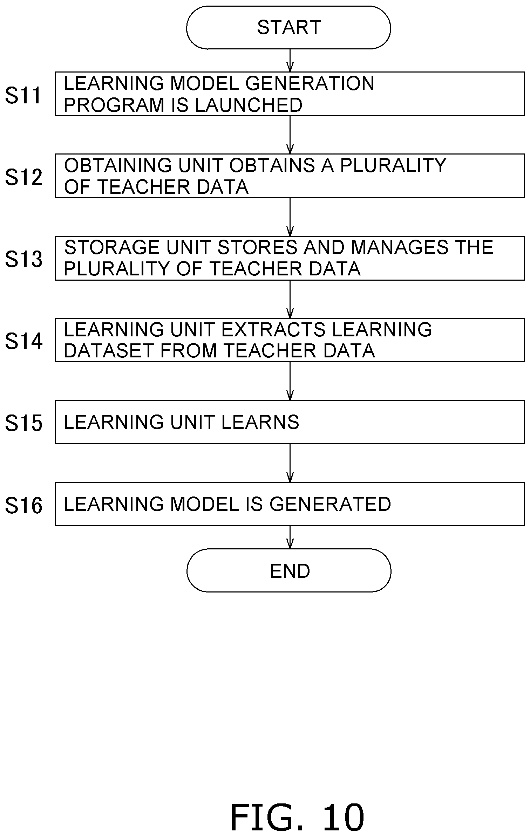

An outline of operation of the learning model generation device 10 is described hereinafter with reference to Figure 10.

First, in step S11, the learning model generation device 10 launches the learning model generation program 15 stored in the storage unit 14. The learning model generation device 10 thus operates on the basis of the learning model generation program 15 to start generating a learning model.

In step S12, the obtaining unit 12 obtains a plurality of teacher data on the basis of the learning model generation program 15.

In step S13, the obtaining unit 12 stores the plurality of teacher data in the database 16 built in the storage unit 14. The storage unit 14 stores and appropriately manages the plurality of teacher data.

In step S14, the learning unit 13 extracts a learning dataset from the teacher data stored in the storage unit 14. An A-dataset to be extracted is determined according to a learning objective of the learning model generated by the learning model generation device 10. The dataset is based on the teacher data.

In step S15, the learning unit 13 learns on the basis of a plurality of datasets thus extracted.

In step S16, the learning model corresponding to the learning objective is generated on the basis of a result of learning by the learning unit 13 in step S15.

The operation of the learning model generation device 10 is thus terminated. Note that sequence and the like of the operation of the learning model generation device 10 can be changed accordingly. The learning model thus generated is: implemented to a general-purpose computer or terminal; downloaded as software or an application; or distributed in a state of being stored in a storage medium, for practical application.

(5) Configuration of the user device 20

Figure 2 shows a configuration of the user device 20 used by a user in the present embodiment. As used herein, the term "user" refers to a person who inputs some information to the user device 20 or causes the user device 20 to output some information. The user device 20 uses the learning model generated by the learning model generation device 10.

The user device 20 is a device having a function of a computer. The user device 20 may include a communication interface such as an NIC and a DMA controller, and be capable of communicating with the learning model generation device 10 and the like through a network. Although the user device 20 shown in Figure 2 is illustrated as one device, the user device 20 is preferably compatible with cloud computing. Consequently, as for a hardware configuration, the user device 20 is not required to be accommodated in one housing or provided as a single device. For example, the user device 20 is configured in such a way that hardware resources thereof are dynamically connected and disconnected according to a load.

The user device 20 includes, for example, an input unit 24, an output unit 25, a control unit 21, and a storage unit 26.

(5-1) Input unit 24

The input unit 24 is, for example, a keyboard, a touch screen, a mouse, and the like. The user can input information to the user device 20 through the input unit 24.

(5-2) Output unit 25

The output unit 25 is, for example, a display, a printer, and the like. The output unit 25 is capable of outputting a result of analysis by the user device 20 using the learning model as well.

(5-3) Control unit 21

The control unit 21 is, for example, a CPU and executes control of the user device 20 as a whole. The control unit 21 includes function units such as an analysis unit 22, an updating unit 23.

The analysis unit 22 of the control unit 21 analyzes the input information being input through the input unit 24, by using the learning model as a program stored in the storage unit 26 in advance. The analysis unit 22 preferably employs the aforementioned machine learning approach for analysis; however, the present disclosure is not limited thereto. The analysis unit 22 can output a correct answer even to unknown input information, by using the learning model having learned in the learning model generation device 10.

The updating unit 23 updates the learning model stored in the storage unit 26 to an optimal state, in order to obtain a high-quality learning model. The updating unit 23 optimizes weighting between neurons in each layer in a neural network, for example.

(5-4) Storage unit 26

The storage unit 26 is an example of the storage medium and constituted of, for example, flash memory, RAM, an HDD, or the like. The storage unit 26 includes the learning model to be executed by the control unit 21, being stored in advance. The storage unit 26 is provided with a database 27 in which a plurality of the teacher data are stored and appropriately managed. Note that, in addition thereto, the storage unit 26 may also store information such as the learning dataset. The teacher data stored in the storage unit 26 is information such as the base material information, the treatment agent information, the evaluation, the environment information as described above.

(6) Operation of user device 20

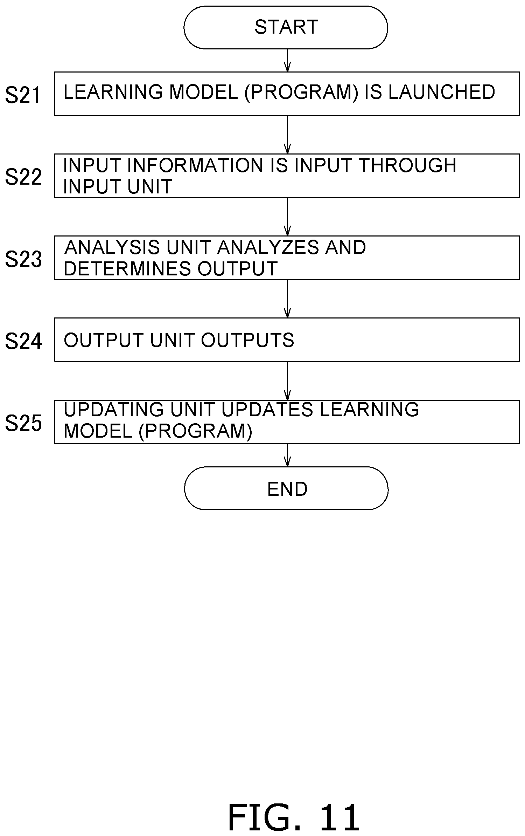

An outline of operation of the user device 20 is described hereinafter with reference to Figure 11. The user device 20 is in such a state that the learning model generated by the learning model generation device 10 is stored in the storage unit 26.

First, in step S21, the user device 20 launches the learning model stored in the storage unit 26. The user device 20 operates on the basis of the learning model.

In step S22, the user who uses the user device 20 inputs input information through the input unit 24. The input information input through the input unit 24 is transmitted to the control unit 21.

In step S23, the analysis unit 22 of the control unit 21 receives the input information from the input unit 24, analyzes the input information, and determines information to be output from the output unit. The information determined by the analysis unit 22 is transmitted to the output unit 25.

In step S24, the output unit 25 outputs result information received from the analysis unit 22.

In step S25, the updating unit 23 updates the learning model to an optimal state on the basis of the input information, the result information, and the like.

The operation of the user device 20 is thus terminated. Note that sequence and the like of the operation of the user device 20 can be changed accordingly.

(7) Specific examples

Hereinafter, specific examples of using the learning model generation device 10 and the user device 20 described above are explained.

(7-1) Water-repellency learning model

In this section, a water-repellency learning model that outputs water-repellency is explained.

(7-1-1) Water-repellency learning model generation device 10

In order to generate the water-repellency learning model, the water-repellency learning model generation device 10 needs to obtain a plurality of teacher data including information regarding at least a type of a base material, a type of a dye with which a surface of the base material is dyed, a type of a monomer constituting a repellent polymer contained in the surface-treating agent, a content of a monomeric unit in the repellent polymer, a content of the repellent polymer in the surface-treating agent, a type of a solvent, a content of the solvent in the surface-treating agent, a type of a surfactant and a content of the surfactant in the surface-treating agent, and water-repellency information. Note that the water-repellency learning model generation device 10 may also obtain other information.

Through learning based on the teacher data thus obtained, the water-repellency learning model generation device 10 can generate the water-repellency learning model that receives as inputs: the base material information including information regarding the type of a base material and the type of a dye with which a surface of the base material is dyed; and the treatment agent information including information regarding the type of a monomer constituting a repellent polymer contained in the surface-treating agent, the content of a monomeric unit in the repellent polymer, the content of the repellent polymer in the surface-treating agent, the type of a solvent, the content of the solvent in the surface-treating agent, and the type of a surfactant and the content of the surfactant in the surface-treating agent, and outputs water-repellency information.

(7-1-2) User device 20 using water-repellency learning model

The user device 20 is capable of using the water-repellency learning model. The user who uses the user device 20 inputs to the user device 20: the base material information including information regarding the type of a base material and the type of a dye with which a surface of the base material is dyed; and the treatment agent information including information regarding the type of a monomer constituting a repellent polymer contained in the surface-treating agent, the content of a monomeric unit in the repellent polymer, the content of the repellent polymer in the surface-treating agent, the type of a solvent, the content of the solvent in the surface-treating agent, and the type of a surfactant and the content of the surfactant in the surface-treating agent.
The user device 20 uses the water-repellency learning model to determine the water-repellency information. The output unit 25 outputs the water-repellency information thus determined.

(7-2) Oil-repellency learning model

In this section, an oil-repellency learning model that outputs oil-repellency is explained.

(7-2-1) Oil-repellency learning model generation device 10

In order to generate the oil-repellency learning model, the oil-repellency learning model generation device 10 needs to obtain a plurality of teacher data including information regarding at least a type of a base material, a type of a dye with which a surface of the base material is dyed, a type of a monomer constituting a repellent polymer contained in the surface-treating agent, a content of a monomeric unit in the repellent polymer, a content of the repellent polymer in the surface-treating agent, a type of a solvent, a content of the solvent in the surface-treating agent, a type of a surfactant and a content of the surfactant in the surface-treating agent, and oil-repellency information. Note that the oil-repellency learning model generation device 10 may also obtain other information.
Through learning based on the teacher data thus obtained, the oil-repellency learning model generation device 10 can generate the oil-repellency learning model that receives as inputs: the base material information including information regarding the type of a base material and the type of a dye with which a surface of the base material is dyed; and the treatment agent information including information regarding the type of a monomer constituting a repellent polymer contained in the surface-treating agent, the content of a monomeric unit in the repellent polymer, the content of the repellent polymer in the surface-treating agent, the type of a solvent, the content of the solvent in the surface-treating agent, and the type of a surfactant and the content of the surfactant in the surface-treating agent, and outputs oil-repellency information.

(7-2-2) User device 20 using oil-repellency learning model

The user device 20 is capable of using the oil-repellency learning model. The user who uses the user device 20 inputs to the user device 20: the base material information including information regarding the type of a base material and the type of a dye with which a surface of the base material is dyed; and the treatment agent information including information regarding the type of a monomer constituting a repellent polymer contained in the surface-treating agent, the content of a monomeric unit in the repellent polymer, the content of the repellent polymer in the surface-treating agent, the type of a solvent, the content of the solvent in the surface-treating agent, and the type of a surfactant and the content of the surfactant in the surface-treating agent.
The user device 20 uses the oil-repellency learning model to determine the oil-repellency information. The output unit 25 outputs the oil-repellency information thus determined.

(7-3) Antifouling property learning model

In this section, an antifouling property learning model that outputs antifouling property is explained.

(7-3-1) Antifouling property learning model generation device 10

In order to generate the antifouling property learning model, the antifouling property learning model generation device 10 needs to obtain a plurality of teacher data including information regarding at least a type of a base material, a type of a dye with which a surface of the base material is dyed, a type of a monomer constituting a repellent polymer contained in the surface-treating agent, a content of a monomeric unit in the repellent polymer, a content of the repellent polymer in the surface-treating agent, a type of a solvent, a content of the solvent in the surface-treating agent, a type of a surfactant and a content of the surfactant in the surface-treating agent, and antifouling property information. Note that the antifouling property learning model generation device 10 may also obtain other information.

Through learning based on the teacher data thus obtained, the antifouling property learning model generation device 10 can generate the antifouling property learning model that receives as inputs: the base material information including information regarding the type of a base material and the type of a dye with which a surface of the base material is dyed; and the treatment agent information including information regarding the type of a monomer constituting a repellent polymer contained in the surface-treating agent, the content of a monomeric unit in the repellent polymer, the content of the repellent polymer in the surface-treating agent, the type of a solvent, the content of the solvent in the surface-treating agent, and the type of a surfactant and the content of the surfactant in the surface-treating agent, and outputs antifouling property information.

(7-3-2) User device 20 using antifouling property learning model

The user device 20 is capable of using the antifouling property learning model. The user who uses the user device 20 inputs to the user device 20: the base material information including information regarding the type of a base material and the type of a dye with which a surface of the base material is dyed; and the treatment agent information including information regarding the type of a monomer constituting a repellent polymer contained in the surface-treating agent, the content of a monomeric unit in the repellent polymer, the content of the repellent polymer in the surface-treating agent, the type of a solvent, the content of the solvent in the surface-treating agent, and the type of a surfactant and the content of the surfactant in the surface-treating agent.

The user device 20 uses the antifouling property learning model to determine the antifouling property information. The output unit 25 outputs the antifouling property information thus determined.

(7-4) Processing stability learning model

In this section, a processing stability learning model that outputs processing stability is explained.

(7-4-1) Processing stability learning model generation device 10

In order to generate the processing stability learning model, the processing stability learning model generation device 10 needs to obtain a plurality of teacher data including information regarding at least a type of a base material, a type of a dye with which a surface of the base material is dyed, a type of a monomer constituting a repellent polymer contained in the surface-treating agent, a content of a monomeric unit in the repellent polymer, a content of the repellent polymer in the surface-treating agent, a type of a solvent, a content of the solvent in the surface-treating agent, a type of a surfactant and a content of the surfactant in the surface-treating agent, and processing stability information. Note that the processing stability learning model generation device 10 may also obtain other information.

Through learning based on the teacher data thus obtained, the processing stability learning model generation device 10 can generate the processing stability learning model that receives as inputs: the base material information including information regarding the type of a base material and the type of a dye with which a surface of the base material is dyed; and the treatment agent information including information regarding the type of a monomer constituting a repellent polymer contained in the surface-treating agent, the content of a monomeric unit in the repellent polymer, the content of the repellent polymer in the surface-treating agent, the type of a solvent, the content of the solvent in the surface-treating agent, and the type of a surfactant and the content of the surfactant in the surface-treating agent, and outputs processing stability information.

(7-4-2) User device 20 using processing stability learning model

The user device 20 is capable of using the processing stability learning model. The user who uses the user device 20 inputs to the user device 20: the base material information including information regarding the type of a base material and the type of a dye with which a surface of the base material is dyed; and the treatment agent information including information regarding the type of a monomer constituting a repellent polymer contained in the surface-treating agent, the content of a monomeric unit in the repellent polymer, the content of the repellent polymer in the surface-treating agent, the type of a solvent, the content of the solvent in the surface-treating agent, and the type of a surfactant and the content of the surfactant in the surface-treating agent.

The user device 20 uses the processing stability learning model to determine the processing stability information. The output unit 25 outputs the processing stability information thus determined.

(7-5) Water-repellent agent learning model

In this section, a water-repellent agent learning model that outputs the optimal water-repellent agent is explained.

(7-5-1) Water-repellent agent learning model generation device 10

In order to generate the water-repellent agent learning model, the water-repellent agent learning model generation device 10 needs to obtain a plurality of teacher data including information regarding at least a type of a base material, a type of a dye with which a surface of the base material is dyed, a type of a monomer constituting a repellent polymer contained in the surface-treating agent, a content of a monomeric unit in the repellent polymer, a content of the repellent polymer in the surface-treating agent, a type of a solvent, a content of the solvent in the surface-treating agent, a type of a surfactant and a content of the surfactant in the surface-treating agent, and water-repellency information. Note that the water-repellent agent learning model generation device 10 may also obtain other information.

Through learning based on the teacher data thus obtained, the water-repellent agent learning model generation device 10 can generate the water-repellent agent learning model that receives as an input the base material information including information regarding the type of a base material and the type of a dye with which a surface of the base material is dyed, and outputs repellent agent information that is optimal for the base material.

(7-5-2) User device 20 using water-repellent agent learning model

The user device 20 is capable of using the water-repellent agent learning model. The user who uses the user device 20 inputs to the user device 20 the base material information including information regarding the type of a base material and the type of a dye with which a surface of the base material is dyed.

The user device 20 uses the water-repellent agent learning model to determine the repellent agent information that is optimal for the base material. The output unit 25 outputs the repellent agent information thus determined.

(7-6) Oil-repellent agent learning model

In this section, an oil-repellent agent learning model that outputs the optimal oil-repellent agent is explained.

(7-6-1) Oil-repellent agent learning model generation device 10

In order to generate the oil-repellent agent learning model, the oil-repellent agent learning model generation device 10 needs to obtain a plurality of teacher data including information regarding at least a type of a base material, a type of a dye with which a surface of the base material is dyed, oil-repellency information, a type of a monomer constituting a repellent polymer contained in the surface-treating agent, a content of a monomeric unit in the repellent polymer, a content of the repellent polymer in the surface-treating agent, a type of a solvent, a content of the solvent in the surface-treating agent, a type of a surfactant and a content of the surfactant in the surface-treating agent, and oil-repellency information. Note that the oil-repellency learning model generation device 10 may also obtain other information.

Through learning based on the teacher data thus obtained, the oil-repellent agent learning model generation device 10 can generate the oil-repellent agent learning model that receives as an input the base material information including information regarding the type of a base material and the type of a dye with which a surface of the base material is dyed, and outputs repellent agent information that is optimal for the base material.

(7-6-2) User device 20 using oil-repellent agent learning model

The user device 20 is capable of using the oil-repellent agent learning model. The user who uses the user device 20 inputs to the user device 20 the base material information including information regarding the type of a base material and the type of a dye with which a surface of the base material is dyed.

The user device 20 uses the oil-repellent agent learning model to determine the repellent agent information that is optimal for the base material. The output unit 25 outputs the repellent agent information thus determined.

(8) Characteristic features

A learning model generation method according to the present embodiment generates a learning model for determining by using a computer an evaluation of an article in which a surface-treating agent is fixed onto a base material. The learning model generation method includes the obtaining step S12, the learning step S15, and the generating step S16. In the obtaining step S12, the computer obtains teacher data. The teacher data includes base material information, treatment agent information, and an evaluation of an article. The base material information is information regarding a base material. The treatment agent information is information regarding a surface-treating agent. In the learning step S15, the computer learns on the basis of a plurality of the teacher data obtained in the obtaining step S12. In the generating step S16, the computer generates the learning model on the basis of a result of learning in the learning step S15. The article is obtained by fixing the surface-treating agent onto the base material. The learning model receives input information as an input, and outputs the evaluation. The input information is unknown information different from the teacher data. The input information includes at least the base material information and the treatment agent information.

The computer uses a learning model, as a program, having further learned the base material information, the treatment agent information, and the evaluation as the teacher data as described above, to determine an evaluation. The learning model includes the input step S22, the determination step S23, and the output step S24. In the input step S22, unknown information different from the teacher data, including the base material information and the treatment agent information, is input. In the determination step S23, the computer uses the learning model to determine the evaluation. In the output step S24, the computer outputs the evaluation determined in the determination step S23.

Conventionally, an article in which a surface-treating agent is fixed to a base material has been evaluated on site by testing every combination of various base materials and surface-treating agents. Such a conventional evaluation method requires extensive time and a considerable number of steps, and there has been a demand for an improved evaluation method.

In addition, as disclosed in Patent Literature 2 (JPA No. 1999-511949), programs and the like employing neural network have been designed for outputting an optimal combination in other fields; however, in the special field of a water-repellent agent, no programs or the like employing neural network have been designed.

The learning model generated by the learning model generation method according to the present embodiment enables evaluation by using a computer. Reduction of the extensive time and the considerable number of steps, which have been conventionally required, is thus enabled. The reduction of the number of steps in turn enables reduction of human resources and cost for the evaluation.

A learning model generation method according to the present embodiment generates a learning model for determining, by using a computer, an optimal surface-treating agent for a base material. The learning model generation method includes the obtaining step S12, the learning step S15, and the generating step S16. In the obtaining step S12, the computer obtains teacher data. The teacher data includes base material information, treatment agent information, and an evaluation. The base material information is information regarding a base material. The treatment agent information is information regarding a surface-treating agent. The evaluation is regarding the article in which the surface-treating agent is fixed onto the base material. In the learning step S15, the computer learns on the basis of a plurality of the teacher data obtained in the obtaining step S12. In the generating step S16, the computer generates the learning model on the basis of a result of learning in the learning step S15. The article is obtained by fixing the surface-treating agent onto the base material. The learning model receives input information as an input, and outputs the evaluation. The input information is unknown information different from the teacher data. The input information includes at least the base material information.

The computer uses a learning model, as a program, having further learned the base material information, the treatment agent information, and the evaluation as the teacher data as described above, to determine treatment agent information. The program includes the input step S22, the determination step S23, and the output step S24. In the input step S22, unknown information different from the teacher data, including the base material information, is input. In the determination step S23, the computer uses the learning model to determine treatment agent information that is optimal for the base material. In the output step S24, the computer outputs the treatment agent information determined in the determination step S23.

With the conventional evaluation method, when a poor-evaluated combination of a base material and a surface-treating agent is found on site, the combination may need research and improvement in a research institution, whereby selection of a surface-treating agent optimal for a substrate requires extensive time and a considerable number of steps.

The learning model generated by the learning model generation method according to the present embodiment enables determination of an optimal surface-treating agent for a base material by using a computer. Time, the number of steps, human resources, cost, and the like required for selecting an optimal surface-treating agent can thus be reduced.

In the learning step S15 of the learning model generation method according to the present embodiment, the learning is preferably performed by a regression analysis and/or ensemble learning that is a combination of a plurality of regression analyses.

The evaluation by the learning model as a program according to the present embodiment is any of water-repellency information, oil-repellency information, antifouling property information, or processing stability information. The water-repellency information is information regarding water-repellency of the article. The oil-repellency information is information regarding oil-repellency of the article. The antifouling property information is information regarding an antifouling property of the article. The processing stability information is preferably information regarding processing stability of the article.

The base material is preferably a textile product.

The base material information includes information regarding at least a type of the textile product and a type of a dye. The treatment agent information includes information regarding at least a type of a monomer constituting a repellent polymer contained in the surface-treating agent, a content of a monomeric unit in the polymer, a content of the repellent polymer in the surface-treating agent, a type of a solvent and a content of the solvent in the surface-treating agent, and a type of a surfactant and a content of the surfactant in the surface-treating agent.

The teacher data includes environment information during processing of the base material. The environment information includes information regarding any of temperature, humidity, curing temperature, or processing speed during the processing of the base material. The base material information preferably further includes information regarding any of a color, a weave, basis weight, yarn thickness, or zeta potential of the textile product. The treatment agent information further includes information regarding any item of: a type and a content of an additive to be added to the surface-treating agent; pH of the surface-treating agent; or zeta potential thereof.

The teacher data preferably includes information regarding many items, and the greater number of pieces as possible of the teacher data is preferred. A more accurate output can thus be obtained.

The learning model as a program according to the present embodiment may also be distributed in a form of a storage medium storing the program.

The learning model according to the present embodiment is a learned model having learned by the learning model generation method. The learned model causes a computer to function to: perform calculation based on a weighting coefficient of a neural network with respect to base material information, which is information regarding the base material, and treatment agent information, which is information regarding a surface-treating agent to be fixed onto the base material, being input to an input layer of the neural network; and output water-repellency information or oil-repellency information of an article from an output layer of the neural network. The weighting coefficient is obtained through learning of at least the base material information, the treatment agent information, and an evaluation of the base material in which the surface-treating agent is fixed onto the base material, as teacher data. The article is obtained by fixing the surface-treating agent onto the base material.

The learned model causes a computer to function to: perform calculation based on a weighting coefficient of a neural network with respect to base material information, which is information regarding the base material, being input to an input layer of the neural network; and to output treatment agent information that is optimal for the base material from an output layer of the neural network. The weighting coefficient is obtained through learning of at least the base material information, the treatment agent information, and an evaluation of the base material onto which the surface-treating agent is fixed, as teacher data. The treatment agent information is information regarding a surface-treating agent to be fixed onto the base material. The article is obtained by fixing the surface-treating agent onto the base material.

The embodiment of the present disclosure has been described in the foregoing; however, it should be construed that various modifications of modes and details can be made without departing from the spirit and scope of the present disclosure set forth in Claims.

[Reference Signs List]

S12    Obtaining step
S15    Learning step
S16    Generating step
S22    Input step
S23    Determination step
S24    Output step

[Citation List]

[Patent Literature]

[Patent Literature 1] JPA No. 2018-535281
[Patent Literature 2] JPA No. 1999-511949

**Claims**

1. A learning model generation method of generating a learning model for determining by using a computer an evaluation of an article in which a surface-treating agent is fixed onto a base material, comprising:

   an obtaining step (S12) in which the computer obtains, as teacher data, information including at least base material information, which is information regarding the base material, treatment agent information, which is information regarding the surface-treating agent, and the evaluation of the article;
   a learning step (S15) in which the computer learns on the basis of a plurality of the teacher data obtained in the obtaining step (S12); and
   a generating step (S16) in which the computer generates the learning model on the basis of a result of learning in the learning step (S15),
   wherein:

   the article is obtained by fixing the surface-treating agent onto the base material;
   the learning model receives input information, which is unknown information different from the teacher data, as an input, and outputs the evaluation; and
   the input information includes at least the base material information and the treatment agent information.

2. A learning model generation method comprising:

   an obtaining step (S12) in which a computer obtains, as teacher data, information including at least base material information, which is information regarding a base material, treatment agent information, which is information regarding a surface-treating agent to be fixed onto the base material, and an evaluation of an article in which the surface-treating agent is fixed onto the base material;
   a learning step (S15) in which the computer learns on the basis of a plurality of the teacher data obtained in the obtaining step (S12); and
   a generating step (S16) in which the computer generates a learning model on the basis of a result of learning in the learning step (S15),
   wherein:

   the article is obtained by fixing the surface-treating agent onto the base material;
   the learning model receives input information, which is unknown information different from the teacher data, as an input, and outputs treatment agent information that is optimal for the base material; and
   the input information includes at least the base material information and information regarding the evaluation.

3. The learning model generation method according to claim 1 or 2, wherein in the learning step (S15), the learning is performed by a regression analysis and/or ensemble learning that is a combination of a plurality of regression analyses.

4. A program with which a computer determines, by using a learning model, an evaluation of an article in which a surface-treating agent is fixed onto a base material, comprising:

   an input step (S22) in which the computer receives input information as an input;
   a determination step (S23) in which the computer determines the evaluation; and
   an output step (S24) in which the computer outputs the evaluation determined in the determination step (S23),
   wherein:

   the article is obtained by fixing the surface-treating agent onto the base material;
   the learning model learns, as teacher data, information including at least base material information, which is information regarding the base material, treatment agent information, which is information regarding the surface-treating agent, and the evaluation; and
   the input information is unknown information different from the teacher data, including at least the base material information and the treatment agent information.

5. A program with which a computer determines, by using a learning model, treatment agent information that is optimal for fixation onto a base material, comprising:

an input step (S22) in which the computer receives input information as an input;
a determination step (S23) in which the computer determines the treatment agent information that is optimal; and
an output step (S24) in which the computer outputs the treatment agent information that is optimal determined in the determination step (S23),
wherein:

the learning model learns, as teacher data, information including at least base material information, which is information regarding the base material, treatment agent information, which is information regarding the surface-treating agent to be fixed onto the base material, and the evaluation of an article in which the surface-treating agent is fixed onto the base material;
the input information is unknown information different from the teacher data, including at least the base material information and information regarding the evaluation; and
the article is obtained by fixing the surface-treating agent onto the base material.

6. The program according to claim 4 or claim 5, wherein the evaluation includes any of:
water-repellency information, which is information regarding water-repellency of the article; oil-repellency information, which is information regarding oil-repellency thereof; antifouling property information, which is information regarding an antifouling property thereof; or processing stability information, which is information regarding processing stability thereof.

7. The program according to any of claims 4 to 6, wherein the base material is a textile product.

8. The program according to claim 7, wherein:
the base material information comprises information regarding at least a type of the textile product and a type of a dye; and
the treatment agent information comprises information regarding at least a type of a monomer constituting a repellent polymer contained in the surface-treating agent, a content of the monomer in the repellent polymer, a content of the repellent polymer in the surface-treating agent, a type of a solvent and a content of the solvent in the surface-treating agent, and a type of a surfactant and a content of the surfactant in the surface-treating agent.

9. The program according to claim 8, wherein:
the teacher data further comprises environment information during processing of the base material;

the environment information comprises information regarding any of concentration of the surface-treating agent in a treatment tank, temperature, humidity, curing temperature, or processing speed during the processing of the base material;
the base material information further comprises information regarding any of a color, a weave, basis weight, yarn thickness, or zeta potential of the textile product; and
the treatment agent information further comprises information regarding any item of: a type and a content of an additive to be added to the surface-treating agent; pH of the surface-treating agent, or zeta potential thereof.

10. A storage medium comprising the program according to any of claims 4 to 9 being stored therein.

11. A learned model for causing a computer to function to perform calculation based on a weighting coefficient of a neural network with respect to base material information and treatment agent information being input to an input layer of the neural network, and to output an evaluation of an article from an output layer of the neural network, wherein:

the weighting coefficient is obtained through learning of at least the base material information, the treatment agent information, and the evaluation as teacher data;
the base material information is information regarding a base material;
the treatment agent information is information regarding a surface-treating agent to be fixed onto the base material;
the evaluation is regarding the article in which the surface-treating agent is fixed onto the base material; and
the article is obtained by fixing the surface-treating agent onto the base material.

12. A learned model for causing a computer to function to perform calculation based on a weighting coefficient of a neural network with respect to base material information and information regarding an evaluation being input to an input layer of the neural network, and to output treatment agent information that is optimal for a base material from an output layer of the neural network, wherein:

the weighting coefficient is obtained through learning of at least the base material information, the treatment agent information, and the evaluation as teacher data;
the base material information is information regarding the base material;
the treatment agent information is information regarding a surface-treating agent to be fixed onto the base material;
the evaluation is regarding an article in which the surface-treating agent is fixed onto the base material; and
the article is obtained by fixing the surface-treating agent onto the base material.

FIG. 1

USER DEVICE

CONTROL UNIT — 21

ANALYSIS UNIT — 22

UPDATING UNIT — 23

INPUT UNIT — 24

OUTPUT UNIT — 25

20

STORAGE UNIT — 26

LEARNING MODEL (PROGRAM)

DATABASE — 27

# FIG. 2

FIG. 3

FIG. 4

FIG. 5

FIG. 6

FIG. 7

FIG. 8

16

| CF3CF2-(CF2CF2)2-CH2CH2OCOC(CH3)=CH2 | Stearyl Acrylate | Polyoxyethylene OleylEther | Temp | Dry Cure | Speed | Bath pH | Processing Concentration | Blocked isocyanate | deciTex | Water Repellency | Oil Repellency |
|---|---|---|---|---|---|---|---|---|---|---|---|
| 16.6 | 2.8 | 1.1 | 23.4 | 170 | 53 | 4.7 | 2.0 | 0.5 | 300 | 90 | 3 |
| 16.8 | 1.4 | 1.3 | 23.4 | 170 | 53 | 4.5 | 4.0 | 0.5 | 300 | 100 | 5 |
| 0 | 27.0 | 1.1 | 25.8 | 170 | 48 | 5.0 | 1.0 | 0.5 | 300 | 80 | 0 |
| 0 | 27.0 | 1.1 | 25.8 | 170 | 48 | 4.8 | 4.0 | 0.5 | 300 | 100 | 0 |

FIG. 9

START

S11 | LEARNING MODEL GENERATION PROGRAM IS LAUNCHED

S12 | OBTAINING UNIT OBTAINS A PLURALITY OF TEACHER DATA

S13 | STORAGE UNIT STORES AND MANAGES THE PLURALITY OF TEACHER DATA

S14 | LEARNING UNIT EXTRACTS LEARNING DATASET FROM TEACHER DATA

S15 | LEARNING UNIT LEARNS

S16 | LEARNING MODEL IS GENERATED

END

# FIG. 10

START

S21 | LEARNING MODEL (PROGRAM) IS LAUNCHED

S22 | INPUT INFORMATION IS INPUT THROUGH INPUT UNIT

S23 | ANALYSIS UNIT ANALYZES AND DETERMINES OUTPUT

S24 | OUTPUT UNIT OUTPUTS

S25 | UPDATING UNIT UPDATES LEARNING MODEL (PROGRAM)

END

# FIG. 11

**INTERNATIONAL SEARCH REPORT**

International application No.

PCT/JP2020/018967

A. CLASSIFICATION OF SUBJECT MATTER

G06F 30/27(2020.01)i; G06N 20/00(2019.01)i; G06N 20/20(2019.01)i; G16C 20/70(2019.01)i
FI: G06F17/50 638; G06N20/00 130; G06N20/20; G16C20/70; G06F17/50 604D

According to International Patent Classification (IPC) or to both national classification and IPC

B. FIELDS SEARCHED

Minimum documentation searched (classification system followed by classification symbols)
G06F30/00-30/28; G06N20/00-20/20; G16C20/70

Documentation searched other than minimum documentation to the extent that such documents are included in the fields searched

| | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2011-180120 A (DAIWA HOUSE INDUSTRY CO., LTD.) 15.09.2011 (2011-09-15) paragraphs [0034]-[0091] | 1-12 |
| A | JP 2008-190880 A (TAIYO KOGYO CORPORATION) 21.08.2008 (2008-08-21) paragraphs [0018]-[0031] | 1-12 |
| A | EP 1146447 A1 (UNILEVER N. V.) 17.10.2001 (2001-10-17) paragraphs [0004]-[0020] | 1-12 |

☐ Further documents are listed in the continuation of Box C.  ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 15 July 2020 (15.07.2020) | 28 July 2020 (28.07.2020) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

Information on patent family members

International application No.

PCT/JP2020/018967

| Patent Documents referred in the Report | Publication Date | Patent Family | Publication Date |
|---|---|---|---|
| JP 2011-180120 A | 15 Sep. 2011 | (Family: none) | |
| JP 2008-190880 A | 21 Aug. 2008 | (Family: none) | |
| EP 1146447 A1 | 17 Oct. 2001 | US 6768973 B1 CA 2323852 A1 | |

Form PCT/ISA/210 (patent family annex) (January 2015)

33

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018535281 A

- JP 1999511949 A